# EUROPEAN PATENT APPLICATION

(11) **EP 4 137 183 A1**
(43) Date of publication of application: **22.02.2023**
(21) Application number: 21840765.8
(22) Date of filing: 07.07.2021
(51) Int. Cl.: A61M 5/44, A61M 5/145, A61M 5/142

(54) **DRUG THERMOSTAT ATTACHABLE/DETACHABLE TO/FROM DRUG PRESSURE INJECTION SYSTEM AND METHOD FOR OPERATING SAME**

(71) Applicant: Impact Korea Co., Ltd., Anyang-si, Gyeonggi-do 14081 (KR)
(72) Inventor: LEE, Myoung Sook, Anyang-si Gyeonggi-do 14103 (KR)
(74) Representative: Schmitt-Nilson Schraud Waibel Wohlfrom Patentanwälte Partnerschaft mbB
(86) International application number: PCT/KR2021/008665
(87) International publication number: WO 2023/282370

(57) **Abstract**

A medicine temperature control device which is attachable to or detachable from a medicine pressure injection system and a driving method thereof are disclosed. A medicine temperature control device which is attachable to or detachable from a medicine pressure injection system according to an exemplary embodiment of the present disclosure includes a pressurization module which includes an expansion unit which expands to pressurize a container containing a medicine, a controller which supplies and pressurizes a fluid to the expansion unit and controls a temperature of the medicine, and a power source which supplies a power to drive the controller; and a temperature control module which includes a temperature control chamber to control a temperature of the medicine.

## Description

### [Technical Field]

The present disclosure relates to a medicine temperature control device attachable to or detachable from a medicine pressure injection system and a driving method thereof.

### [Background Art]

When a patient is transported from a disaster scene such as a traffic/mountain accident site or a battlefield, in order to increase the survival probability of the patient, medicine injection should be carried out preemptively and quickly at the scene. At this time, a device which controls a temperature of the medicine is necessary to inject a medical fluid having the same temperature as the human body temperature.

A medicine having a temperature similar to a normal body temperature of the human needs to be injected into a patient having a low body temperature. Further, with respect to a hot environment or a patient having a fever, it is necessary to control the temperature to be lower. However, currently, only a device which warms a medicine is used, but development of a product which may control the temperature is insufficient.

Further, a device of the related art in which a medicine injecting unit and a warming unit are integrally formed is configured by a controller to inject a medicine, a driving unit which controls a medicine injecting speed and pressure, a warming unit to warm the medicine, and a power source operated by a battery so that it is disadvantageous due to a large volume and a high manufacturing cost.

A medical device in which a medicine injecting device and a warming unit are integrally formed is being used in hospitals as well as emergency medical fields in the disaster scene. Actually, in the hospitals, even though the medicine warming is not necessary, the expensive integrated type device is purchased to be used.

A related art of the present disclosure is disclosed in Korean Registered Patent Publication No. 10-1835820.

### [Disclosure]

### [Technical Problem]

The present disclosure is provided to solve the above-described problems of the related art and an object thereof is to provide a technique which directly connects an expansion unit which directly pressurizes a container containing a medicine with a medicine injecting device and integrally or separately uses the medicine injecting device and a temperature control chamber depending on a situation.

However, objects to be achieved by various embodiments of the present disclosure are not limited to the technical objects as described above and other technical objects may be present.

### [Technical Solution]

As a technical means to achieve the above-described technical object, according to an aspect of the present disclosure, a medicine temperature control device which is attachable to or detachable from a medicine pressure injection system includes a pressurization module which includes an expansion unit which expands to pressurize a container containing a medicine, a controller which supplies and pressurizes a fluid to the expansion unit and controls a temperature of the medicine, and a power source which supplies a power to drive the controller; and a temperature control module which includes a temperature control chamber to control a temperature of the medicine.

Further, the pressurization module and the temperature control module may be provided to be attachable to each other or detachable from each other.

Further, the temperature control module may be supplied with a power to heat from the power source in a state in which the pressurization module and the temperature control module are coupled.

Further, the temperature control module may include a cartridge in which the medicine supplied from the container stays to be heated before being injected to a subject and a thermoelectric element disposed to transfer heat to the cartridge.

Further, the temperature control chamber may be disposed to accommodate the thermoelectric element therein and enclose the cartridge.

Further, the controller may control the fluid to be supplied at a pressurized pressure corresponding to the injection pressure to inject the medicine into the subject based on a predetermined injection pressure.

Further, the controller may control the temperature control module to inject the medicine into the subject based on a predetermined injection temperature.

Further, the temperature control module may include: an inlet side injection passage through which the medicine is supplied from the container to the cartridge; an outlet side injection passage through which the heated medicine is injected into the subject from the cartridge; and at least one temperature sensor disposed to be adjacent to at least one of the inlet side injection passage and the outlet side injection passage.

Further, the controller may control the thermoelectric element based on the measured temperature received from the temperature sensor.

Further, the medicine pressure injection system including a temperature control module according to an exemplary embodiment of the present disclosure may include an output unit which outputs at least one of the injection pressure, the pressurized pressure, the injection temperature, and the measured temperature.

Further, the output unit may be provided for the pressurization module.

Further, the output unit may output remaining power information of the power source.

Further, the power source may include a plurality of battery cells.

Further, a battery pack including the plurality of battery cells or each of the plurality of battery cells may be provided to be attachable to or detachable from the pressurization module.

In the meantime, according to another aspect of the present disclosure, a pressurization module of a medicine pressure injection system may include a main body; an expansion unit which expands to pressurize a container containing a medicine; a pressurization unit which supplies a fluid to the expansion unit; and a power source which supplies a power to drive the pressurization unit and includes a plurality of battery cells provided to be attachable to or detachable from the main body.

In the meantime, according to another aspect of the present disclosure, a temperature control module of a medicine pressure injection system may include a cartridge in which the supplied medicine stays to be heated before being injected to a subject; a thermoelectric element which transfers heat to the cartridge; an inlet side injection passage through which the medicine is supplied from the container containing the medicine to the cartridge; an outlet side injection passage through which the heated medicine is injected into the subject from the cartridge; and at least one temperature sensor disposed to be adjacent to at least one of the inlet side injection passage and the outlet side injection passage.

In the meantime, according to another aspect of the present disclosure, a driving method of a medicine temperature control device which is attachable to or detachable from a medicine pressure injection system may include (a) setting an injection pressure and an injection temperature of a medicine; (b) controlling a pressurization module to pressurize a container containing the medicine with a pressurized pressure corresponding to the injection pressure; and (c) controlling the temperature control module provided to heat the medicine based on the injection temperature.

Further, the driving method of a medicine temperature control device which is attachable to or detachable from a medicine pressure injection system according to an exemplary embodiment of the present disclosure may include supplying a power for at least one of the pressurization and the heating from the power source in a state in which the pressurization module and the temperature control module are coupled.

The above-described solving means are merely illustrative but should not be construed as limiting the present disclosure. In addition to the above-described embodiments, additional embodiments may be further provided in the drawings and the detailed description of the present disclosure.

### [Advantageous Effects]

According to the solving means of the present disclosure, a technique which directly connects an expansion unit which directly pressurizes a container containing a medicine with a medicine injecting device and integrally or separately uses the medicine injecting device and a temperature control chamber depending on a situation may be provided.

According to the solving means of the present disclosure, a medicine temperature control device which is attachable to or a detachable from a medicine pressure injecting system in which a pressurization module which automatically pressurizes a container containing a medicine using a pump, etc. to inject the medicine into a body of a subject and a temperature control module which warms the injected medicine at a predetermined temperature are integrally provided to allow a prompt treatment at the emergency scene and a driving method thereof may be provided.

However, the effect which may be achieved by the present disclosure is not limited to the above-described effects, there may be another effect.

### [Description of Drawings]

FIGS. 1 and 2 are schematic diagrams of a medicine pressure injection system including a temperature control module according to an exemplary embodiment of the present disclosure.
FIG. 3 is a conceptual view for explaining a function and an operation of a pressurization module.
FIG. 4 is a view illustrating a pressurization module including two or more pressurization units.
FIG. 5 is a view illustrating a power source of a pressurization module.
FIG. 6 is a view illustrating a placement structure of a cartridge and a thermoelectric element of a temperature control module.
FIGS. 7A and 7B are views illustrating a fixing unit which supports and fixes a medicine pressure injection system including a temperature control module according to an exemplary embodiment of the present disclosure to an external structure.
FIG. 8 is an operational flowchart of a driving method of a medicine pressure injection system including a temperature control module according to an exemplary embodiment of the present disclosure.

### [Best Mode]

Hereinafter, the present disclosure will be described more fully hereinafter with reference to the accompanying drawings, in which exemplary embodiments of the present disclosure are shown. However, the present disclosure may be realized in various different forms, and is not limited to the embodiments described herein. Accordingly, in order to clearly explain the present disclosure in the drawings, portions not related to the description are omitted. Like reference numerals designate like elements throughout the specification.

Throughout this specification and the claims that follow, when it is described that an element is "coupled" to another element, the element may be "directly coupled" to the other element or "electrically coupled" or "indirectly coupled" to the other element through a third element.

Through the specification of the present disclosure, when one member is located "on", "above", "on an upper portion", "below", "under", and "on a lower portion" of the other member, the member may be adjacent to the other member or a third member may be disposed between the above two members.

In the specification of the present disclosure, unless explicitly described to the contrary, the word "comprise" and variations such as "comprises" or "comprising", will be understood to imply the inclusion of stated elements but not the exclusion of any other elements.

The present disclosure relates to a medicine temperature control device attachable to or detachable from a medicine pressure injection system and a driving method thereof. For example, the present disclosure relates to a medicine pressure injection system which pressures a container containing a medicine to quickly inject a large amount of medicine into a body of a subject in an emergency scene and warms the medicine to be injected and a driving method thereof.

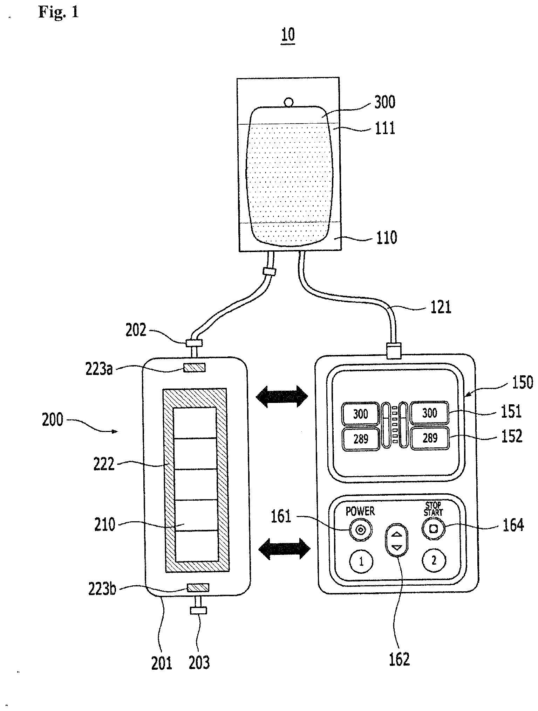

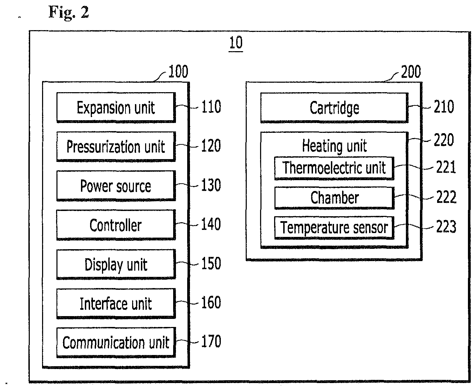

FIGS. 1 and 2 are schematic diagrams of a medicine pressure injection system including a temperature control module according to an exemplary embodiment of the present disclosure.

Referring to FIGS. 1 and 2, a medicine injection system 10 (hereinafter, simply referred to as an "injection system 10") which is attachable to or detachable from a medicine pressure injection system according to an exemplary embodiment of the present disclosure may include a pressurization module 100 and a temperature control module 200.

Further, even though not illustrated in the drawings, at least one of the pressurization module 100 and the temperature control module 200 of the injection system 10 and a user terminal (not illustrated) of a medical staff, etc. may communicate with each other via a network (not illustrated). The network (not illustrated) means a connection structure which allows information exchange between nodes such as terminals or servers. Examples of the network (not illustrated) include 3^{rd} generation partnership project (3GPP) network, a long term evolution (LTE) network, a 5G network, a world interoperability for microwave access (WIMAX) network, Internet, a local area network (LAN), a wireless local area network (Wireless LAN), a wide area network (WAN), a personal area network (PAN), a Wi-Fi network, a Bluetooth network, a satellite broadcasting network, an analog broadcasting network, a digital multimedia broadcasting (DMB) network, and the like, but are not limited thereto.

In the meantime, for example, a user terminal (not illustrated) which is held by a medical staff who manipulates the injection system 10 and interworks with the pressurization module 100 and the temperature control module 200 may include all kinds of wireless communication devices such as a smart phone, a smart pad, a tablet PC, a personal communication system (PCS), a global system for mobile communication (GSM), a personal digital cellular (PDC), a personal handphone system (PHS), a personal digital assistant (PDA), an international mobile telecommunication (IMT)-2000, code division multiple access (CDMA)-2000, W-code division multiple access (W-CDMA), and a wireless broadband internet (Wibro) terminal, but is not limited thereto.

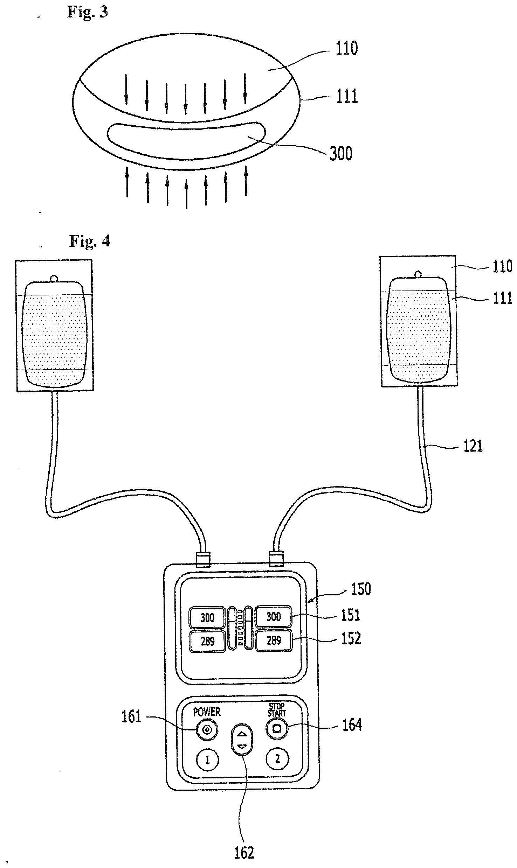

Referring to FIGS. 2 and 3, the pressurization module 100 may include an expansion unit 110, a pressurization unit 120, a power source 130, and a controller 140. Further, the pressurization module 100 may further include at least one of a display unit 150, an interface unit 160, and a communication unit 170.

Further, the pressurization module 100 may include a main body 101 in which sub components such as the pressurization unit 120 and the power source 130 are mounted. Desirably, the main body 101 may be configured to stably accommodate the subcomponents such as the pressurization unit 120 and the power source 130 described above, but a characteristic such as a shape or a material is not limited to any one characteristic, but may be designed in various forms depending on the implementation embodiment of the present disclosure.

Specifically, the pressurization module 100 may include the expansion unit 110 which expands to pressurize a container 300 containing a medicine, the pressurization unit 120 which supplies a fluid to the expansion unit 110, and the power source 130 which supplies a power to drive the pressurization unit 120.

In the meantime, in the description of the exemplary embodiment of the present disclosure, the "medicine" is a liquid which may be injected through a patient's blood vessel and may be a term which comprehensively refers to fluids, physiological saline solutions, Ringer's solutions, nutrients, intravenous fluids. Further, a medicine which is injected into the body of the subject by means of the injection system 10 disclosed in the present disclosure according to an implementation embodiment of the present disclosure may broadly include blood, electrolyte solutions, blood-like medicines, and specific medicines which may be mixed with the medicine to be injected.

Further, the container 300 containing the medicine may also be referred to as a bag, a pouch, or the like.

Further, according to an exemplary embodiment of the present disclosure, the pressurization unit 120 includes a pump (not illustrated) which supplies a predetermined pressure to supply a fluid to the expansion unit 110, a fluid supply pipe 121 through which the fluid supplied to the expansion unit 110 passes, and a pressure sensor (not illustrated) which measures an operating pressure of the pump (not illustrated). In the meantime, the fluid supply pipe 121 may be provided to be attachable to or detachable from the main body 101.

The controller 140 may control the pressurization unit 120 to supply the fluid to the expansion unit 110 at a pressurized pressure corresponding to a predetermined injection pressure so as to inject the medicine contained in the container 300 into a subject based on the predetermined injection pressure.

For example, the controller 140 may appropriately control an operating pressure of the pump (not illustrated) to supply the fluid to the expansion unit 110 at an appropriate pressurized pressure from the pressurization unit 120 based on an injection pressure which is set by a user input applied by an interface unit 160 to be described below or set to a predetermined pressure level in association with the driving of the pressurization module 100.

In the meantime, the expansion unit 110 may be provided to increase the volume by the fluid (for example, air, etc.) supplied by the operating pressure of the pump (not illustrated) of the pressurization unit 120. Further, the expansion unit 110 may be formed to have a structure sealed to endure the pressurized pressure to an external direction generated by the fluid supplied thereinto.

Further, the support unit 111 may be a component disposed such that both ends are fixed to the expansion unit 110 so that the container 300 containing the medicine is interposed between the support unit 111 and the expansion unit 110.

According to the exemplary embodiment of the present disclosure, the expansion unit 110 and the support unit 111 and the container 300 may be integrally provided, but are not limited thereto so that depending on the implementation embodiment of the present disclosure, the expansion unit 110 and the support unit 111 and the container 300 may be individually provided.

Further, according to an exemplary embodiment of the present disclosure, the support unit 111 may be formed of a light transmitting material (for example, a transparent material, a translucent material, or the like) to allow the user to visually identify information marked on an outer surface of the container 300 containing the medicine and/or a remaining amount of the medicine contained therein, but is not limited thereto.

FIG. 3 is a conceptual view for explaining a function and an operation of a pressurization module.

Referring to FIG. 3, the container 300 containing the medicine is located between the expansion unit 110 and the support unit 111. When the fluid (for example, air, etc.) is injected to the expansion unit 110 by the pressurization unit 120 so that the expansion unit 110 expands, the pressure is applied to the container 300 in the direction of the arrow illustrated in FIG. 3. Therefore, a predetermined pressurized pressure is applied to the container 300 and the medical fluid contained in the container 300 is injected into the subject by the pressurized pressure in response to the predetermined injection pressure.

FIG. 4 is a view illustrating a pressurization module including two or more pressurization units.

Referring to FIG. 4, the pressurization module 100 may be designed to include a plurality of pressurization units 120 depending on the implementation embodiment of the present disclosure. For reference, the pressurization module 100 including the single pressurization unit 120 described above with reference to FIG. 1 is referred to as a single type pressurization module and a pressurization module 100 including two distinguishable pressurization units 120 which may be understood with reference to FIG. 4 may be referred to as a dual type pressurization module.

Further, referring to FIG. 4, a dual type pressurization module or a pressurization module including two or more pressurization units may individually include a fluid supply pipe 121, a display unit 151, and an interface unit 160 corresponding to each of the plurality of pressurization units 120 in the main body 101.

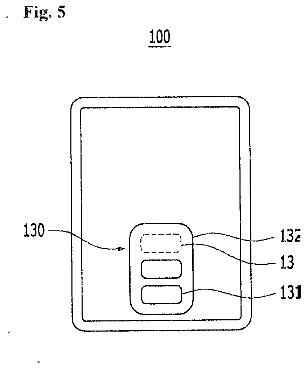

FIG. 5 is a view illustrating a power source of a pressurization module.

Referring to FIG. 5, the power source 130 of the pressurization module 100 may include a plurality of battery cells 131. Further, according to an exemplary embodiment of the present disclosure, the power source 130 may include a battery pack 132 including a plurality of battery cells 131.

According to an exemplary embodiment of the present disclosure, each of the battery cells 131 may be a lithium-ion battery, but is not limited thereto. The battery cell provided in the injection system 10 disclosed in the present disclosure may be provided as various battery types which have been conventionally developed or will be developed in the future.

Further, referring to FIG. 5, each battery cell 131 of the power source 130 or the battery pack 132 including the plurality of battery cells 131 may be provided to be attachable to or detachable from the pressurization module 100. For example, the attachable battery cell 131 or battery pack 132 of the power source 130 may be provided to be inserted into a mounting groove 13 formed on a rear surface of the main body 101 of the pressurization module 100 or to be separated from a combined state from the mounting groove 13, but is not limited thereto. Therefore, according to the implementation embodiment of the present disclosure, a region to which the battery cell 131 or the battery pack 132 is coupled may be set to various positions such as a lower surface or an upper surface of the main body 101.

Hereinafter, a temperature control module 200 of the injection system 10 will be described.

The temperature control module 200 may include a heating unit 220 provided to heat the medicine which is injected into the body of the subject and a cartridge 210 in which the medicine supplied from the container 300 stays to be heated by the heating unit 220 before injecting the medicine into the subject.

Further, the temperature control module 200 may include an inlet side injection passage 202 through which the medicine is supplied from the container 300 to the cartridge 210 and an outlet side injection passage 203 through which the heated medicine is injected from the cartridge 210 to the subject. Various members (a transfer tube, catheter, etc.) for transferring the medicine may be connected to the inlet side injection passage 202 and the outlet side injection passage 203 and a shape or a size of the member for transferring the medicine may be determined in various forms depending on the implementation embodiment of the present disclosure.

Further, the temperature control module 200 may include at least one temperature sensor 223 disposed to be adjacent to at least one of the inlet side injection passage 202 and the outlet side injection passage 203.

With regard to this, desirably, the temperature control module 200 may include a first temperature sensor 223a and a second temperature sensor 223b to measure a temperature of a medicine before being heated by the heating unit 220 by means of the first temperature sensor 223a which measures the temperature of the medicine to be supplied from the container 300 to the cartridge 210 and subsequently measure a temperature of the medicine which is heated (warmed) by the heating unit 220 by means of the second temperature sensor 223b which measures a temperature of the medicine which is discharged to be injected into the body of the subject from the cartridge 210.

Further, the heating unit 220 may include the thermoelectric element 221 disposed to transfer heat to the medicine contained in the cartridge 210. According to the exemplary embodiment of the present disclosure, the thermoelectric element 221 may be a Peltier module, but is not limited thereto.

Further, the heating unit 220 may include a chamber 222 disposed to accommodate the above-described thermoelectric element 221 therein and enclose the cartridge 210 in which the medicine stays. Further, according to the exemplary embodiment of the present disclosure, the chamber 222 disposed to enclose the cartridge 210 may be provided with a member having heat conservation and heat insulation characteristics to quickly warm the medicine by reducing the dissipation of the heat generated by the thermoelectric element 221 to the outside of the cartridge 210 or provided with a heating member such as a heat wire mounted therein to transfer additional heat to the medicine as well as the heat generated by the thermoelectric element 221.

In the meantime, referring to FIG. 1, the pressurization module 100 and the temperature control module 200 of the injection system 10 disclosed in the present disclosure may be provided to be attachable to each other or detachable from each other. Further, the heating unit 220 of the temperature control module 200 may be supplied with the power (for example, a power to flow a predetermined current to the thermoelectric element 221, etc.) to heat the medicine from the power source 130 provided in the pressurization module 100 in a state in which the pressurization module 100 and the temperature control module 200 are coupled to each other.

With regard to this, in the description of the exemplary embodiment of the present disclosure, the state in which the pressurization module 100 and the temperature control module 200 are coupled (attached) to each other may be a concept including not only a state in which a main body 101 of the pressurization module 100 and a main body 201 of the temperature control module 200 are mechanically coupled, but also a state in which a sub component of the pressurization module 100 and a sub component of the temperature control module 200 are electrically connected.

In other words, in the state in which the pressurization module 100 and the temperature control module 200 are coupled (attached) to each other, an electrical operation between the pressurization module 100 and the temperature control module 200 may be performed by supplying a power to drive the heating unit 220 of the temperature control module 200 by the power source 130 or transmitting a measurement value of the temperature sensor 223 provided in the heating unit 220 of the temperature control module 200 to the controller 140 of the pressurization module 100.

With regard to this, a pair of fastening members which may be mechanically coupled to each other may be provided on outer surfaces of the main body 101 of the pressurization module 100 and the main body 201 of the temperature control module 200. For example, a protrusion member which outwardly protrudes is formed on the outer surface of the main body 101 of the pressurization module 100 and a groove member which is inwardly dented is formed on the outer surface of the main body 201 of the temperature control module 200. In a state in which the protrusion member and the groove member are disposed by the user to be in contact with each other, an external force is applied to couple the protrusion member and the groove member to each other to mechanically attach the pressurization module 100 and the temperature control module 200. However, the present disclosure is not limited thereto and the pressurization module 100 and the temperature control module 200 may be coupled by utilizing various connection (coupling) mechanisms which may be employed in the field of the mechanical tool.

Further, the pair of fastening members may be provided with a material which allows bi-directional electric-conduction between the pressurization module 100 and the temperature control module 200 at the time of coupling so that the sub component of the pressurization module 100 and the sub component of the temperature control module 200 are electrically connected by the coupling of the pair of fastening members.

Further, according to the exemplary embodiment of the present disclosure, the controller 140 of the pressurization module 100 may control the heating unit 220 of the temperature control module 200 based on a predetermined injection temperature to inject the medicine based on the predetermined injection temperature. For example, the controller 140 may control the heating unit 220 by adjusting a current amount applied to the thermoelectric element 221 based on a deviation of a measured temperature received from the temperature sensor 223 of the temperature control module 200 and the predetermined injection temperature, but is not limited thereto.

Further, according to the exemplary embodiment of the present disclosure, the injection system 10 may include an output unit (not illustrated) which outputs at least one of the predetermined injection pressure, an actual pressurized pressure applied to the expansion unit 110 by driving the pump (not illustrated), a predetermined injection temperature, and a measured temperature sensed by the temperature sensor 223.

For example, the output unit (not illustrated) may include at least one of an acoustic unit (not illustrated) which audibly outputs the above-described various information and a display unit 150 which visually output the various information.

Further, the pressurization module 100 may include an interface unit 160 which receives a user input associated with the driving control of the injection system 10.

According to the exemplary embodiment of the present disclosure, the interface unit 160 may include various sub modules which are capable of receiving user manipulation inputs, such as a power controller 161 which is provided to allow a user who manipulates the injection system 10 to turn on or off the driving of the pressurization unit 120, a pressure controller 162 which controls a pressure (a setting pressure) at which the fluid is supplied to the container 300 by the pressurization unit 120, a display controller (not illustrated) which determines a type of information to be displayed on the display unit 150, and an emergency controller 164 which stops the driving of the pressurization unit 120 and discharges the fluid injected to the expansion unit 110 by the pressurization unit 120. However, desirably, it is understood that the sub modules of the interface unit 160 are examples. Therefore, according to the implementation embodiment of the present disclosure, various user input acquiring means which allow the user to perform various manipulations associated with the pressurization module 100 or the temperature control module 200 are also provided in the interface unit 160.

According to the exemplary embodiment of the present disclosure, the display unit 150 may display a setting pressure which is set by a user input applied by the interface unit 160 or set to a basic pressure level which is associated with the driving of the pressurization module 100 to be set in advance and an operating pressure of the pump measured by the pressure sensor (not illustrated). Further, according to the exemplary embodiment of the present disclosure, the display unit 150 may display pressure information which is actually applied to the container 300 to be sensed by the expansion unit 110 and an elapsed time (an operating time) from a time at which an operation of at least one of the pressurization module 100 and the temperature control module 200 starts. For example, a type of information displayed on the display unit 150 may be determined in response to the user input applied to the display controller (not illustrated).

In the meantime, for example, the display unit 150 may include a first display unit 151 which displays a setting pressure and a second display unit 152 which displays an operating pressure of the pump measured at a corresponding timing.

Further, according to the exemplary embodiment of the present disclosure, the output unit (not illustrated) may be provided for the pressurization module 100. Further, the output unit (not illustrated) may output remaining power information of the power source 130.

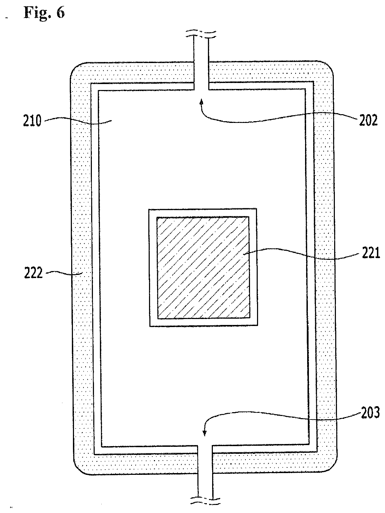

FIG. 6 is a view illustrating a placement structure of a cartridge and a thermoelectric element of a temperature control module.

Referring to FIG. 6, the cartridge 210 of the temperature control module 200 may include a hollow portion in which the thermoelectric element 221 is disposed to increase an area or a volume of an inner region of the cartridge 210 to which a heat generated by the thermoelectric element 221 is transmitted so that the medicine quickly reaches a predetermined injection temperature.

Further, the hollow portion of the cartridge 210 in which the thermoelectric element 221 is disposed may be located to be close to a center portion of the cartridge 210 so as to relatively uniformly transfer the heat which is transferred to the medicine contained in the cartridge 210 to a region in which the medicine is contained.

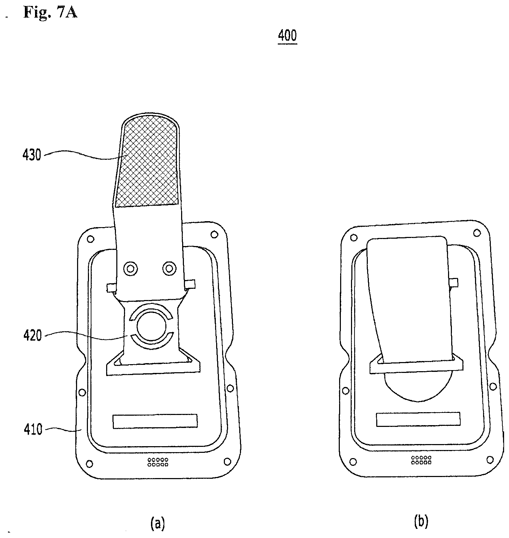

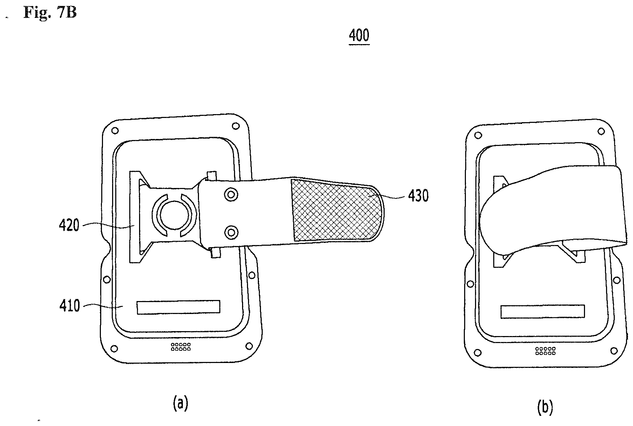

FIGS. 7A and 7B are views illustrating a fixing unit which supports and fixes a medicine pressure injection system including a temperature control module according to an exemplary embodiment of the present disclosure to an external structure.

Referring to FIGS. 7A and 7B, the fixing unit 400 is provided with respect to the main body 101 of the pressurization module 100 and/or the main body 201 of the temperature control module 200 to fix the pressurization module 100 and/or the temperature control module 200 to an external structure (for example, a columnar structure, etc.). As another example, the fixing unit 400 may use a part of the body (for example, an arm, a leg, or the like) of the subject (patient) as a support structure to fix the main body 101 or 201. With regard to this, even in the emergency scene in which it is difficult to provide a columnar structure such as a pole and the subject (patient) needs to be quickly transported, the patient may be treated by stably supporting the pressurization module 100 and the temperature control module 200 using the fixing unit 400.

Specifically, referring to FIGS. 7A and 7B, the fixing unit 400 may include a support base 410, a rotary bracket 420 which is rotatably provided along a plane direction of the support base 410 on a top surface of the support base 410 and includes a pair of ring shaped members, and a belt member 430 having one end which is fixed to any one of the pair of ring shaped members of the rotary bracket 420 and the other end which is inserted to pass through the other one of the pair of ring shaped members of the rotary bracket 420 in a state in which the external structure is disposed with respect to the fixing unit 400.

Further, according to the exemplary embodiment of the present disclosure, an anti-slip material such as rubber or polyurethane is applied or a pattern shape is configured on an inner surface of the belt member 430 to increase a frictional force against a surface of the external structure.

To be more specific, FIG. 7A illustrates that the pressurization module 100 and/ or the temperature control module 200 is fixed to the external structure (for example, a vertical pole, etc.) extending in a vertical direction by means of the fixing unit 400. FIG. 7B may illustrate that the pressurization module 100 and/or the temperature control module 200 is fixed to the external structure (for example, a horizontal pole, etc.) extending in a horizontal direction by means of the fixing unit 400.

With regard to this, the rotary bracket 420 of the fixing unit 400 is provided to be rotatable along a plane direction of the support base 410 so that the main body (in other words, the main body 101 of the pressurization module 100, the main body 201 of the temperature control module 200, or the like) of the injection system 10 may be operated to be supported with respect to various external structures without being tilted, in active response to various on-site environments, which increases the convenience of the user.

Further, according to the exemplary embodiment of the present disclosure, the rotary bracket 420 is not only rotatable so as to correspond only to the horizontal direction or the vertical direction as illustrated in FIGS. 7A and 7B, but also rotates at 360 degrees along the plane direction of the support base 410 so that the pressurization module 100 and/or the temperature control module 200 of the injection system 10 may be effectively fixed to the structure which is obliquely disposed, other than the vertical structure and the horizontal structure.

In the meantime, according to the exemplary embodiment of the present disclosure, the fixing unit 400 may be coupled to an intermediate bracket (not illustrated) which is primarily coupled to an outer circumferential surface of the external structure to be supported to more firmly fix the injection system 10 to the external structure.

In the meantime, in the above description, even though an exemplary embodiment that the pressurization module 100 and the temperature control module 200 are coupled to each other to be operated has been mainly described, depending on the implementation embodiment of the injection system 10 disclosed in the present disclosure, the pressurization module 100 and the temperature control module 200 may be provided so as to individually operate in a separated state. For example, the temperature control module 200 may further include a power connecting unit (not illustrated) in which an external power source or a separate battery is mounted in addition to the fastening member for coupling with the pressurization module 100.

Hereinafter, an operation flow of the present disclosure will be described in brief based on the above detailed description.

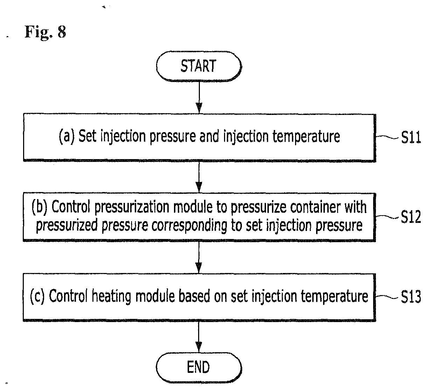

FIG. 8 is an operational flowchart of a driving method of a medicine pressure injection system including a temperature control module according to an exemplary embodiment of the present disclosure.

The driving method of the medicine pressure injection system including a temperature control module illustrated in FIG. 8 may be performed by the above-described injection system 10. Therefore, even though some contents are omitted, the contents which have been described for the injection system 10 may be applied to the description for the driving method of a medicine pressure injection system including a temperature control module in the same way.

Referring to FIG. 8, in step S11, the controller 140 may set an injection pressure and an injection temperature of a medicine (a). According to the exemplary embodiment of the present disclosure, in step S11, the controller 140 may determine at least one setting value of the injection pressure and the injection temperature based on a user input applied by the interface unit 160.

Next, in step S12, the controller 140 may control the pressurization module 100 to pressurize the container 300 containing the medicine with a pressurized pressure corresponding to the set injection pressure (b). According to the exemplary embodiment of the present disclosure, in step S12, the controller 140 may set an operating pressure of the pump (not illustrated) of the pressurization unit 120 to apply the pressurized pressure corresponding to the set injection pressure to the container 300.

Next, in step S13, the controller 140 may control the temperature control module 200 which is provided to heat the medicine based on the predetermined injection temperature (c). According to the exemplary embodiment of the present disclosure, in step S13, the controller 140 may control the temperature control module 200 by determining whether the pressurization module 100 and the temperature control module 200 are coupled (attached) and controlling a current level which is passed through the thermoelectric element 221 of the temperature control module 200 based on a deviation of a measured temperature received from the temperature sensor 223 of the temperature control module 200 and a predetermined injection temperature.

In the above description, steps S11 to S13 may be further divided into additional steps or combined as smaller steps depending on an implementation embodiment of the present disclosure. Further, some steps may be omitted if necessary and the order of steps may be changed.

The driving method of the medicine pressure injection system including the temperature control module according to the exemplary embodiment of the present disclosure may be implemented in the form of a program instruction which may be executed by various computer means to be recorded in a computer readable medium. The computer readable medium may include solely a program instruction, a data file, and a data structure or a combination thereof. The program instruction recorded in the medium may be specifically designed or constructed for the present disclosure or known to those skilled in the art of a computer software to be used. Examples of the computer readable recording medium include a magnetic media such as a hard disk, a floppy disk, or a magnetic tape, an optical media such as a CD-ROM or a DVD, a magneto-optical media such as a floptical disk, and a hardware device which is specifically configured to store and execute the program instruction, such as a ROM, a RAM, and a flash memory. Examples of the program instruction include not only a machine language code which is created by a compiler but also a high level language code which may be executed by a computer using an interpreter. The hardware device may operate as one or more software modules in order to perform the operation of the present disclosure and vice versa.

Further, the driving method of the medicine pressure injection system including the temperature control module may also be implemented as a computer program or an application executed by a computer which is stored in a recording medium.

The above description of the present disclosure is illustrative only and it is understood by those skilled in the art that the present disclosure may be easily modified to another specific type without changing the technical spirit of an essential feature of the present disclosure. Thus, it is to be appreciated that the embodiments described above are intended to be illustrative in every sense, and not restrictive. For example, each component which is described as a singular form may be divided to be implemented and similarly, components which are described as a divided form may be combined to be implemented.

The scope of the present disclosure is represented by the claims to be described below rather than the detailed description, and it is to be interpreted that the meaning and scope of the claims and all the changes or modified forms derived from the equivalents thereof come within the scope of the present disclosure.

## Claims

1. A medicine temperature control device which is attachable to or detachable from a medicine pressure injection system, comprising:
a pressurization module which includes an expansion unit which expands to pressurize a container containing a medicine, a controller which supplies and pressurizes a fluid to the expansion unit and controls a temperature of the medicine, and a power source which supplies a power to drive the controller; and
a temperature control module which includes a temperature control chamber to control a temperature of the medicine.

2. The medicine temperature control device of claim 1, wherein the pressurization module and the temperature control module are provided to be attachable to each other or detachable from each other and the temperature control module is supplied with a power to heat from the power source in a state in which the pressurization module and the temperature control module are coupled.

3. The medicine temperature control device of claim 1, wherein the temperature control module includes a cartridge in which the medicine supplied from the container stays to be heated before being injected to a subject and a thermoelectric element disposed to transfer heat to the cartridge.

4. The medicine temperature control device of claim 3, wherein the temperature control chamber is disposed to accommodate the thermoelectric element therein and enclose the cartridge.

5. The medicine temperature control device of claim 3, wherein the controller controls the fluid to be supplied at a pressurized pressure corresponding to the injection pressure to inject the medicine into the subject based on a predetermined injection pressure.

6. The medicine temperature control device of claim 5, wherein the controller controls the temperature control module to inject the medicine into the subject based on a predetermined injection temperature.

7. The medicine temperature control device of claim 6, wherein the temperature control module includes:
an inlet side injection passage through which the medicine is supplied from the container to the cartridge;
an outlet side injection passage through which the heated medicine is injected into the subject from the cartridge; and
- at least one temperature sensor disposed to be adjacent to at least one of the inlet side injection passage and the outlet side injection passage, and the controller controls the thermoelectric element based on the measured temperature received from the temperature sensor.

8. The medicine temperature control device of claim 7, further comprising:
- an output unit which outputs at least one of the injection pressure, the pressurized pressure, the injection temperature, and the measured temperature.

9. The medicine temperature control device of claim 8, wherein the output unit is provided for the pressurization module.

10. The medicine temperature control device of claim 9, wherein the output unit further outputs remaining power information of the power source.

11. The medicine temperature control device of claim 1, wherein the power source includes a plurality of battery cells and a battery pack including the plurality of battery cells or each of the plurality of battery cells is provided to be attachable to or detachable from the pressurization module.

12. A pressurization module of a medicine pressure injection system, comprising:
- a main body;
- an expansion unit which expands to pressurize a container containing a medicine;
- a pressurization unit which supplies a fluid to the expansion unit; and
- a power source which supplies a power to drive the pressurization unit and includes a plurality of battery cells provided to be attachable to or detachable from the main body.

13. A temperature control module of a medicine pressure injection system, comprising:
- a cartridge in which a supplied medicine stays to be heated before being injected to a subject;
- a thermoelectric element which transfers heat to the cartridge;
- an inlet side injection passage through which the medicine is supplied from a container containing the medicine to the cartridge;
- an outlet side injection passage through which the heated medicine is injected into the subject from the cartridge; and
- at least one temperature sensor disposed to be adjacent to at least one of the inlet side injection passage and the outlet side injection passage.

14. A driving method of a medicine temperature control device which is attachable to or detachable from a medicine pressure injection system, comprising:
- (a) setting an injection pressure and an injection temperature of a medicine;
- (b) controlling a pressurization module to pressurize a container containing the medicine with a pressurized pressure corresponding to the injection pressure; and
- (c) controlling the temperature control module provided to heat the medicine based on the injection temperature.

15. The driving method of claim 14, wherein the pressurization module and the temperature control module are provided to be attachable to each other or detachable from each other and the pressurization module includes a power source,
- supplying a power for at least one of the pressurization and the heating from the power source in a state in which the pressurization module and the temperature control module are coupled is further included.
